Europäisches Patentamt

European Patent Office

Office européen des brevets

(19)

(11)  **EP 0 668 759 B1**

(12)  **EUROPEAN PATENT SPECIFICATION**

(45) Date of publication and mention
of the grant of the patent:
**28.05.2003  Bulletin 2003/22**

(51) Int Cl.[7]: **A61K 9/70**, A61K 31/40

(21) Application number: **94901329.6**

(22) Date of filing: **09.11.1993**

(86) International application number:
**PCT/US93/10761**

(87) International publication number:
**WO 94/010986 (26.05.1994 Gazette 1994/12)**

(54) **TRANSDERMAL DELIVERY OF KETOROLAC**

TRANSDERMALE VERABREICHUNG VON KETOROLAC

APPORT TRANSDERMIQUE DE KETOROLAC

(84) Designated Contracting States:
**CH DE ES FR GB IT LI NL SE**

(30) Priority: **09.11.1992  US 973801
22.01.1993  US 8814
25.01.1993  US 10256**

(43) Date of publication of application:
**30.08.1995  Bulletin 1995/35**

(73) Proprietor: **Neurogesx, Inc.
San Carlos, CA 94070-4117 (US)**

(72) Inventors:
• **MOHR, Judy M.
Menlo Park, CA 94025 (US)**
• **BAKER, Richard W.
Palo Alto, CA 94031 (US)**
• **NAKAJI, Lisa A.
San Jose, CA 95136 (US)**

(74) Representative: **Bizley, Richard Edward
Hepworth, Lawrence, Bryer & Bizley
Merlin House
Falconry Court
Baker's Lane
Epping Essex CM16 5DQ (GB)**

(56) References cited:
US-A- 4 089 969        US-A- 4 910 205
US-A- 5 069 909

• **CHEMICAL ABSTRACTS, vol. 120, no. 6, 7
February 1994, Columbus, Ohio, US; abstract
no. 62322, & WO-A-93 24114 (HISAMITSU
PHARM. CO. INC.,JP) 9 December 1993**
• **CHEMICAL ABSTRACTS, vol. 118, no. 18, 3 May
1993, Columbus, Ohio, US; abstract no. 175803,
& JP-A-04 321 624 (HISAMITSU PHARM. CO.
INC.,JP) 11 November 1992**
• **DATABASE WPI Week 9312, Derwent
Publications Ltd., London, GB; AN 93-100639
(12) & WO-A-93 04677 (HISAMITSU PHARM. CO.
LTD.,JP) 18 March 1993**
• **Journal of Medicinal Chemistry, Volume 29, No.
4, issued 1986, A. GUZMAN et al., "Absolute
Configuration of
(-)-5-Benzoyl-1,2-dihydro-3H-pyrrolo(1,2-a)
pyrrole-1-carboxylic Acid, the Active
Enentiomer of Ketorolac", pages 589-591.**
• **Chemical & Engineering News, issued 28
September 1992, S.C. STINSON et al., "Chiral
Drugs", pages 46-79, see especially page 47.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to the transdermal delivery of the active enantiomer of a chiral drug. More particularly, the invention concerns transdermal delivery of the active enantiomer of ketorolac from a patch that can deliver a therapeutically effective dose of ketorolac or (-) ketorolac through the skin of a patient in need of treatment with ketorolac for an extended period of time of at least 12 hours or more.

## BACKGROUND OF THE INVENTION

Ketorolac is a non-steroidal anti-inflammatory agent with potent analgesic properties. The drug is currently administered as the racemic mixture orally or by injection and is commercially available in forms suited for such modes of delivery. Ketorolac tromethamine salt in sterile water for intramuscular and intravenous administration is available at concentrations ranging from 1.5% (15 mg in 1 ml) to 3% (60 mg in 2 mls). Typically, when injected, a bolus dose of 30 to 60 mg is first given followed by subsequent injections of half the loading dose (15 to 30 mg) every 6 to 8 hours. The total daily doses of the drug as such is in the range of 60-120 mg. Delivered at these levels, the drug is extremely effective. However, the need for repeated injections due to the relatively rapid metabolism of the drug makes this mode of delivery inconvenient in certain situations.

A far more convenient and acceptable form of delivery is simple oral delivery 2 to 3 times per day. However, oral administration of ketorolac can be quite irritating to the gastrointestinal tract. Thus, for oral use, the FDA has approved only low-dosage tablets containing only 10 mg of ketorolac tromethamine salt. Of course patients can take more than one tablet, but in general it is normally not safely possible to maintain the same highly effective blood levels obtained with the injectable form when the drug is given orally.

Thus there is an interest in developing alternative modes of delivering ketorolac which do not have the gastrointestinal side effects produced by oral formulations but which are more convenient than injection methods. Nasal formulations of ketorolac are described in application USSN 875,700, filed 29 April 1992. In copending application USSN 07/973,801 filed 9 November 1992, the transdermal delivery of the active enantiomer of ketorolac is described.

The use of transdermal patches for drug delivery is particularly beneficial when it is desired to maintain a constant blood level of drug in the patient for extended periods of time. However, due to the high dose of ketorolac required for therapeutic efficacy, the estimated transdermal dose required is 30 - 60 mg/day. The transdermal patch size required to deliver this much ketorolac far too large, approximately 2 - 4 $m^2$.

Document US 4910205 discusses transdermal delivery of loratadine in a volatile solvent containing essential oils and fatty acid esters, but teaches nothing about enhancing transdermal delivery of ketorolac.

US 5069909 describes a laminated composite for administering buprenorphine transdermally, optionally in the presence of a permeation enhancer. There is no teaching regarding transdermal delivery of ketorolac.

A paper by Stinson et al in Chemical & Engineering News, issued 28th September 1992, pages 46 to 79 is a review of chiral drug technology and suggests that transdermal delivery of e.g. nicotine might benefit from the advantages of using one isomer instead of a racemic mixture. A table on page 65 lists the "best candidates for racemic switches" but the table does not include ketorolac.

A paper by Guzman et al in the Journal of Medicinal Chemistry, Volume 29 (1986) pages 589 to 591 discusses the relative activity of ketorolac enantiomeres and discusses that the (-)enantiomer is the active isomer.

Accordingly, it is an object of this invention to provide a transdermal patch that can deliver ketorolac at a rate that attains a therapeutic level.

Another object of the present invention is to provide a ketorolac transdermal patch that is less than 30 $cm^2$ in active surface area.

It is a further object of the invention to provide a transdermal ketorolac patch that avoids or minimizes skin irritation.

Another object of the invention is to provide a ketorolac transdermal patch that is effective in providing analgesia for periods of 12 hours or more.

These and other objects and features of the invention will be apparent to those skilled in the art from the following detailed description and appended claims when taken in conjunction with the figures.

## SUMMARY OF THE INVENTION

A patch for the transdermal administration of ketorolac or (-) ketorolac is described. The patch is capable of delivering therapeutically effective levels of ketorolac or (-) ketorolac through a patient's skin for a period of 12 hours or more. The patch comprises an occlusive backing layer having a skin-facing side; and a drug depot having a skin-facing side and a skin-distal side in contact with said skin-facing side of said backing layer, said depot comprising ketorolac or (-) ketorolac, and an enhancer combination in which said ketorolac or (-) ketorolac is dispersed, said enhancer combination increasing the permeability of said ketorolac or (-) ketorolac through the stratum corneum and comprising a plasticizing-type enhancer and a solvent-type enhancer, said plasticizing type enhancer being a member selected from the group consisting of isopropyl myristate, caprylic triglyceride, capric triglyceride and glyceryl oleate, and said solvent type enhancer being a member selected from the group consisting of ethanol, propanol propylene glycol, butanol and glycerin. The patch is capable of delivering ketorolac through a patient's skin at flux rates of about 40 $\mu$g/cm$^2$ hr or more or (-) ketorolac at a flux rate of about 20 $\mu$g/cm$^2$ hr or more.

In a further aspect, the invention provides the use of an occlusive backing layer having a skin-facing side, and means defining a drug depot having a skin-distal side in contact with said skin-facing side of said backing layer, said drug depot containing ketorolac or (-) ketorolac and an enhancer combination for increasing the permeability of said ketorolac or (-) ketorolac through the stratum corneum, said enhancer combination comprising a plasticizing-type enhancer and a solvent-type enhancer, said plasticizing type enhancer being a member selected from the group consisting of isopropyl myristate, caprylic triglyceride, capric triglyceride and glyceryl oleate, and said solvent type enhancer being a member selected from the group consisting of ethanol, propanol, propylene glycol, butanol and glycerin in the manufacture of a medicament in the form of a transdermal patch having a skin-facing side capable of delivering, through a patient's skin, ketorolac at a flux rate of about 40 $\mu$g/cm$^2$ hr or more, e.g. to deliver less than 60 mg of ketorolac or less than 30 mg (-) ketorolac over a 24 hour period; optionally said patch having an active surface area of less than 30 cm$^2$.

In another embodiment of the invention, the transdermal patch is an adhesive matrix type wherein the drug depot is an adhesive polymer in which the (-) ketorolac and enhancer are dispersed.

In another embodiment of the invention, the transdermal patch is a reservoir type whereby the drug depot is defined by the backing layer and a porous membrane which are sealed together at their peripheral edges.

In another embodiment of the present invention, the transdermal patch is a monolithic matrix type wherein the drug depot is a polymer matrix in which the (-) ketorolac and enhancer are dispersed. A porous membrane covers the depot at it skin-facing side, and an adhesive layer covers the skin-facing side of the membrane.

A method of administering ketorolac is also described. The method comprises applying a transdermal patch to the skin of a patient in need of ketorolac, said patch containing ketorolac and an enhancer, and delivering ketorolac through the patient's skin at a flux rate of 40 $\mu$g/cm2 hr or more.

In another embodiment of the present invention, the method comprises applying a transdermal patch to the skin of a patient in need of ketorolac, said patch containing (-) ketorolac and an enhancer and delivering (-) ketorolac through the patient's skin at a flux rate of about 20 $\mu$g/cm$^2$ hr or more.

A further understanding of the nature and advantages of the invention will become apparent by reference to the remaining portions of the specification and drawings.

## BRIEF DESCRIPTION OF THE DRAWINGS

Figure 1 compares the ratio of drug delivered transdermally (controlled release) to the drug delivered as an instant dose (drug injection) required to achieve 24 hour therapy as a function of the half-life of the drug.

Figure 2 shows the relationship between the flux rate of various drugs and their melting points.

Figure 3 shows an adhesive matrix transdermal patch having an impermeable backing and an analgesic-containing adhesive polymer matrix.

Figure 4 shows a reservoir type transdermal patch having an impermeable backing, a drug depot, a porous membrane, and an adhesive layer. The backing and the porous membrane are sealed at their peripheral edges, thus defining the drug depot.

Figure 5 shows a monolithic matrix transdermal patch having an impermeable backing, a drug depot comprising a polymer matrix in which drug is dispersed, an optional porous membrane, and an adhesive layer.

Figure 6 shows a transdermal patch having an impermeable backing, a monolithic analgesic-containing matrix, a porous membrane, and a peripheral adhesive layer that is in contact with a non-active surface area.

## DETAILED DESCRIPTION OF SPECIFIC EMBODIMENTS

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described. For purposes of the present invention, the foregoing terms are defined below.

The term "active surface area" refers to the area of the patch where drug is transmitted to the skin. Accordingly inactive surface area may, for example, be peripheral to the active surface area and may provide an adhesive or structural function.

The term "clearance" as used herein refers to the rate of removal of a drug from the body expressed as the *in vivo* volume equivalent of the substance being removed per unit time. The concept of clearance is described in detail in Rowland & Tozer's, Clinical Pharmacokinetics: Concepts and Applications, (2nd Ed. 1989), which is hereby incorporated by reference. Clearance does not indicate how much drug is being removed from the system but, rather, the volume of biological fluid such as blood or plasma that would have to be completely freed of drug to account for the elimination. Clearance is expressed as a volume per unit of time. Clearance by means of various organs of elimination is additive. Elimination of drug may occur as a result of processes that occur in the kidney, liver, and other organs. When the respective clearance by each organ is added together, they equal total systemic clearance.

The term "ketorolac" as used herein refers to any therapeutic form of the analgesic ketorolac including the free acid and its pharmaceutically effective salts, such as hydrochloride, mesylate, hydrobromide, tromethamine, and the like.

The term "(-) ketorolac" as used herein refers to the active enantiomer of ketorolac free acid and its pharmaceutically effective salts, the active enantiomer being substantially free of the inactive enantiomer.

The term "racemic ketorolac" (or the racemate) as used herein refers to the active and inactive enantiomers of ketorolac free acid in combination and their pharmaceutically effective salts.

The term "therapeutically effective level" as used herein refers to the minimal blood level of drug required to achieve a therapeutic effect.

The use of transdermal patches for drug delivery is particularly beneficial when it is desired to maintain a constant blood level of drug in the patient for extended periods of time. There is an added benefit in that oftentimes, the required dose of a drug when delivered by a 24 hour transdermal patch can be one half or less that of the dose delivered by a single once a day intravenous or oral dose. This is particularly true if the drug has a high clearance value. With conventional drug delivery methods, if a drug has a high clearance value it is necessary to administer a large dose of the drug to extend the time it takes the blood drug levels to fall below the therapeutically effective level. But with transdermal delivery, the dose of a drug with a high clearance value can be lower since the drug is controlled released, and does not have to be administered at levels much greater than the therapeutically effective level.

The half-life of a drug is the amount of time it takes the total level of drug in a body to decrease by 50%. Clearance is related to the half-life of a drug by Equation 1:

$$T\,1/2 = \frac{0.693 \; x \; Volume \; of \; distribution}{Clearance}$$

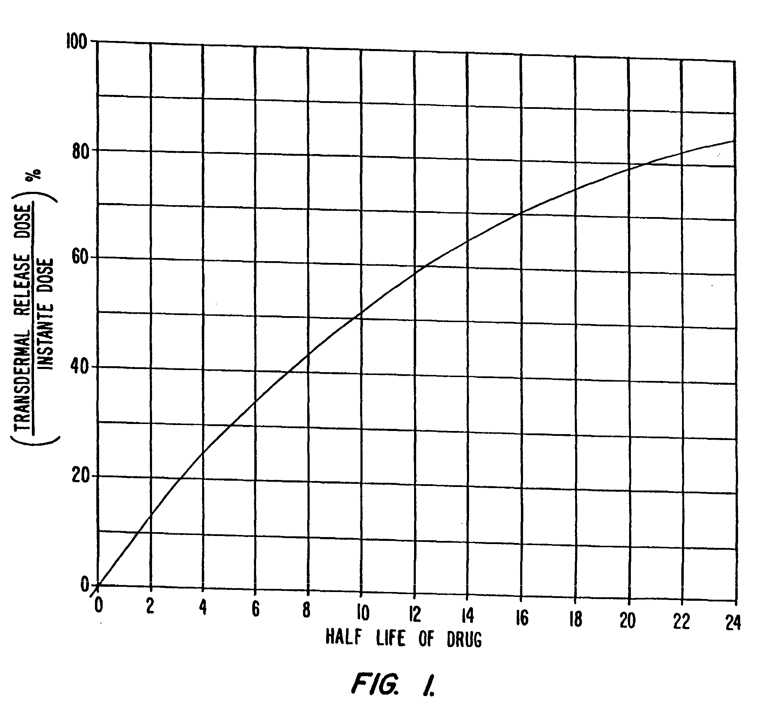

The relationship between the conventional dose of a drug and the dose of drug delivered transdermally can be calculated if the half-life of the drug is known. This procedure is described on pages 5-10 of Baker, R.W., Controlled Release of Biologically Active Agents, John Wiley and Sons, New York, (1987) [hereinafter Baker], which is hereby incorporated by reference. Using this procedure, the data shown in Table I and Figure 1 has been calculated.

Figure 1 is a graphical illustration of the relationship between the conventional dose of a drug and the dose of drug delivered transdermally, where the half life of the drug is known. For example, the x-axis of Figure 1 represents the half life of the drug in question. The point on the y-axis corresponding to that half life is the % ratio between the transdermal release dose and the instant dose of the drug. The instant dose refers to a dosage that is not a controlled release dosage. The most common instant dosage is an intravenous dose. Intramuscular or oral doses are also instant doses, although the blood levels of the drug are subject to an initial delay which varies depending on the half-life of the drug, the method of formulation and the route of administration of the drug.

As noted from the shape of the curve in Figure 1, as the half life of a drug increases, the amount required to be delivered transdermally approaches that required by routes of administration that are not rate-controlled. Illustrations of this relationship between the non-transdermal and the transdermal dose are provided in Table I.

Table I shows that the ratio of dose required for the transdermal delivery of a drug compared to the dose required for conventional drug delivery decreases as the half-life of the drug decreases. Thus, if the half-life of the drug is 24 hours, then a transdermal patch delivering drug to the body at a relatively constant rate should only have to deliver 84.5% of the dose of a conventional instant dose form of the drug delivered once every 24 hours. In this case, the advantage offered by controlled transdermal delivery is relatively small, only a 15.5% reduction in dose. However, if the half-life of the drug is 4 hours, the approximate half-life of ketorolac, then the advantage offered by constant delivery is much greater compared to conventional delivery given once every 24 hours, namely a reduction in dose of almost 75%. Even if an injectable dose is given every 8 hours, the reduction in dose obtained when a transdermal patch is used is still substantial, being on the order of 40 to 50%.

Table I

| Drug Half-life (in hours | Ratio of dose required of controlled release form compared to conventional form (for a 24-hour device) |
|---|---|
| 24 | 0.845 |
| 12 | 0.595 |
| 6 | 0.350 |
| 4 | 0.250 |
| 3 | 0.205 |

Thus it follows that if the total amount of ketorolac delivered by regular injections three times a day to control pain is approximately 60-120 mg/day, then a 30-60 mg/day dose delivered transdermally would produce approximately the same benefit without the problems of overdosing and underdosing associated with injectable delivery. This type of calculation has led to interest in delivering ketorolac transdermally. For example, transdermal delivery of ketorolac from nearly saturated solutions in various enhancer combinations has been studied (D. Yu et al., *Pharm. Res.* 5(7): 457-462, (1988). In another study, a 2% ketorolac topical gel was studied (R. Greenwald, *Drugs of Today,* 29(1): p. 52, (1992)). The patients applied 3 g of the gel three times per day without occlusion. Serum concentrations of approximately 0.17 - 0.18 µg/ml were attained, significantly below the generally accepted target level for good analgesia with ketorolac of 0.3 to 5.0 µg/ml.

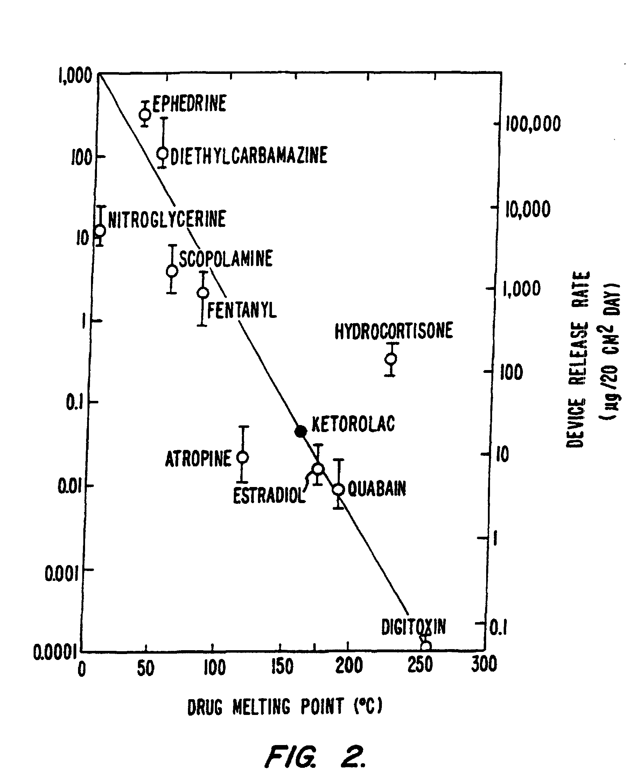

These dissatisfying results are not surprising. It is known in the art of transdermal drug delivery that it is very difficult to deliver drugs at a rate of greater than 10-20 $\mu$g/cm$^2$·hr. Of the 9 drugs delivered by approved commercial transdermal formulations only two, nicotine and nitroglycerine, which are both very permeable liquids, deliver drug at this rate. Most of the other formulations deliver the drugs at a much lower rate. It has been proposed that the permeability of skin to a given drug can be correlated with the drug's melting point according to the relationship set forth in Figure 2 and as described in Baker, *supra.* Based on this, the expected flux of ketorolac with a melting point of 160°C would be 0.06 $\mu$g/cm$^2$·hr. The expected flux of ketorolac tromethamine, which has melting point similar to that of the free acid, would be about the same.

It follows that if the estimated transdermal dose of 30-60 mg/day of ketorolac is to be delivered by a transdermal device, the area of the patch required would be impossibly large, on the order of 2-4 m$^2$. Of course, skin permeation enhancers could be used to increase the delivery, but if conventional sized patches of the order of 30 cm$^2$ or less are to be used, skin permeation rates from the patch of the order of 42-84 $\mu$g/cm$^2$·hr would be required - a very considerable degree of enhancement.

Patch design is governed by several factors: permeability of drug to the skin, dose of drug required, enhancers used to deliver drug, and the flux rate of drug and enhancer required to achieve a therapeutic effect. Accordingly, there is much literature on the design of transdermal patches, with variations on a particular design being influenced by the above factors. Liquid reservoir patches of various designs are known to researchers in the field of transdermal drug delivery. For example, U.S. Patent No. 4,460,372 ('372) discloses a transdermal patch having, in order from skin-distal side of patch to skin-facing side of patch, a backing layer, an enhancer reservoir layer containing a solvent type enhancer such as ethanol, a diffusion membrane layer, and a drug reservoir-contact adhesive layer. In this patent, a rate-controlling membrane separates an enhancer reservoir from the drug depot rather than having the enhancer and drug within the same compartment. The premise is that the drug delivered does not need the rate-controlling effect of the diffusion membrane layer, whereas the enhancer does. Without the diffusion membrane layer, the flux of the enhancer would be too high. However, if the drug were to pass through the diffusion membrane layer, its flux would be too low to achieve a therapeutic effect.

In U.S. Patent No. 4,379,454, a patch somewhat similar to the '372 patch is described. The difference with this patch is that both the drug and the solvent enhancer are contained in the reservoir layer and are separated from the skin by a microporous membrane. The membrane is quite permeable to the drug which is able to permeate the membrane at a rate higher than the skin's ability to absorb the drug. However, the membrane is relatively less permeable to the enhancer. As a result, the enhancer permeates the membrane, and subsequently the skin, at a rate less than the skin's ability to absorb the enhancer. The membrane is thus a rate controlling barrier for the enhancer. When this type of patch is used to deliver drug, the rate of absorption of the drug can be controlled mostly by the permeability of the membrane to the enhancer. If the membrane is relatively impermeable to the enhancer, the rate of delivery of enhancer to the skin will be low and the enhancement effect achieved will also be low. This will result in a low drug absorption rate. Correspondingly if the permeability of the membrane to enhancer is high, the rate of delivery of enhancer to the skin will also be high as well as the resulting enhancement effect achieved. This will result in a high drug absorption rate. The difficulty with these types of devices is that they require precise control of the permeability of the enhancer through the membrane to achieve good control of drug absorption. In practice, this level of control is difficult to achieve.

U.S. Patent No. 4,031,894 describes another type of transdermal patch having an impermeable backing layer, a layer of drug gelled in mineral oil, a microporous membrane, and a contact adhesive layer containing drug. The drug in the contact adhesive layer is the "pulse dosage" which rapidly permeates through the patient's skin. Thereafter the delivery rate of the drug is controlled by the microporous membrane.

European patent application 0 413 487 A1 discloses a transdermal patch for the delivery of dexmedetomidine. It comprises (from skin-distal to skin-facing side) a backing layer, an adhesive layer, a porous intermediate layer, a drug/contact adhesive layer, and a release liner. The porous intermediate layer functions as structural reinforcement and can be made of a non rate-controlling, nonwoven fabric such as polyester. Upon fabrication, the anchor adhesive and contact adhesive migrate into the intermediate layer.

To date, it has been difficult to transdermally deliver a therapeutically effective dose of ketorolac. This is due primarily to the low flux of ketorolac and the large dose required to attain therapeutic blood levels. The applicants however, have discovered that (-) ketorolac is as permeable to human skin as the racemate. This is an important result because the great majority of the pharmaceutical effect of ketorolac is due to the (-) enantiomer of ketorolac while the (+) enantiomer is relatively ineffective. The use of the (-) enantiomer rather than a racemic mixture thus reduces the amount of drug delivered transdermally that is required to equal the therapeutic effect of the conventional bolus formulation. As described above, approximately 30 to 60 mg/day of the racemic mixture of ketorolac is required to achieve the same therapeutic effect as the bolus injectable dose. The transdermal dose of the racemic mixture requires a delivery rate of 40 to 80 $\mu$g/cm$^2$·hr for a conventional 30 cm$^2$ patch. However, transdermal delivery of the (-) enantiomer reduces the required daily dose to 15-30 mg/day or even less. Thus, the required transdermal delivery rate is reduced to 20-40 $\mu$g/cm$^2$·hr from a 30 cm$^2$ patch.

The ketorolac transdermal patches of the present invention result in a skin flux rate of 40-100 $\mu$g/cm$^2$·hr. Thus, they can be used to deliver therapeutically useful amounts of the racemic form of ketorolac. The patches are also suitable for transdermal delivery of the (-) enantiomer of ketorolac which requires a skin flux of approximately 20-40 $\mu$g/cm$^2$·hr. The patches of the present invention, for the delivery of both the (-) enantiomer and racemic form of ketorolac, are therapeutically useful at active surface areas of less than 30 cm$^2$.

Ketorolac tromethamine is freely soluble in water and methanol. It has a pKa of 3.54 and a molecular weight of 376.41. The compound is an off-white, crystalline material that melts between 160°C and 170°C. As such, it is a relatively difficult drug to deliver transdermally because of the extremely lipophilic barrier of the stratum corneum layer of the skin. For adequate skin penetration rates, a chemical enhancer is necessary. There are numerous possible penetration enhancers that can be used such as solvent-type and plasticizing-type enhancers. As used herein, the term "enhancer" is meant to encompass any enhancer or combination of enhancers that increases the permeability of ketorolac to the stratum corneum.

As used herein, "plasticizing-type enhancer" refers to fatty acid esters and similar hydrophobic compounds generally having a molecular weight of greater than 150 but less than 300 and having a water solubility of less than 300 and having a water solubility of less than 0.5 wt% and preferably 0.2 wt% or less. Without limiting the scope of the present invention, the following is proposed as the mechanism of action of plasticizing-type enhancers. The stratum corneum layer of the skin, although only 25-50 microns thick, is the principal barrier to transdermal permeation. It is believed that the function of these plasticizing enhancers is to migrate into the upper stratum corneum layers and to remain there for a prolonged period of time. The migrated plasticizing enhancers serve to increase the mobility and solubility of ketorolac into the skin. To be effective as a plasticizing enhancer, the molecule should be relatively large. In general a molecular weight of approximately 150 is required, or greater than 10 carbon atoms. The plasticizing enhancer must also be relatively water insoluble or it will leach into the subcutaneous tissue layers below the stratum corneum. Thus, plasticizing enhancers with water solubility of less than 0.5 wt% are preferred. It is also preferred that the molecular weight of the plasticizing enhancer be less than 500, because molecules larger than this will have difficulty migrating into the stratum corneum layers. caprylic/capric triglyceride, glyceryl oleate.

As used herein, "solvent-type enhancer" refers to ethanol, propanol, butanol, glycerin, and propylene glycol. These are better enhancers because they generally provide higher flux rates than plasticizing enhancers. These compounds are all relatively low molecular weight, generally less than 150 in molecular weight. Preferred solvent enhancers have a molecular weight of less than 100. They are also relatively hydrophilic, generally being greater than 2 wt% soluble in water, and are preferably greater than 10 wt% soluble in water. Most preferred solvent enhancers are completely water miscible. While these solvent enhancers can be useful in delivering ketorolac through the skin when used alone, large amounts must be applied continuously to get a prolonged therapeutic effect. As such, they are often irritating to the skin. For example, a 60% aqueous solution of the solvent-type enhancer ethanol is extremely irritating to most patients. For most patients, a 40% aqueous solution is generally acceptable. Unfortunately a 40% solution of ethanol is not a sufficiently powerful enhancer for ketorolac.

When a plasticizing type enhancer is used in combination with a solvent type enhancer it is possible to deliver ketorolac through the stratum corneum at therapeutically effective levels. Such a mixture achieves high delivery rates of the drug with relatively dilute solutions of the solvent enhancer. This eliminates the irritation that occurs when solvent-type enhancer solutions are used alone at high concentrations without plasticizing enhancers. When used with plasticizing type enhancers, it is believed that the function of the solvent enhancer is to rapidly diffuse into the stratum corneum layer of the skin making it possible for the heavier, less mobile plasticizing enhancer to enter the stratum corneum layer. The small size and hydrophilic nature of these solvent enhancers makes them very effective in this role. However, these molecules tend to migrate through the stratum corneum layers and into the subcutaneous tissues very quickly.

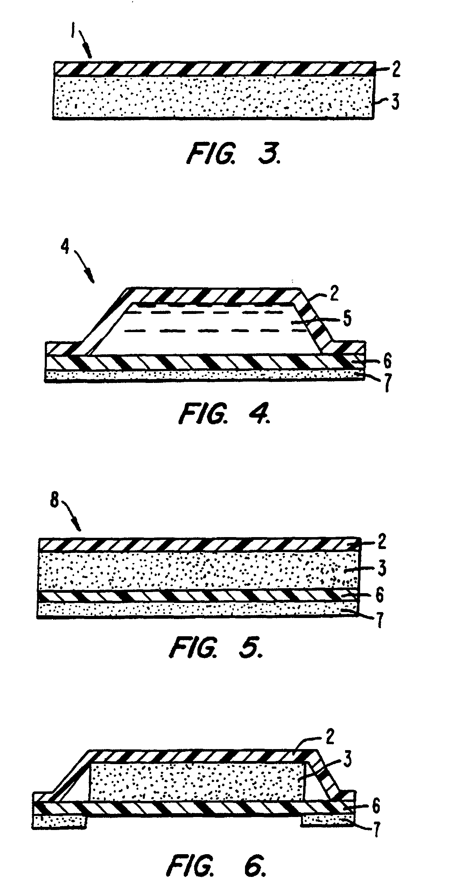

There are several designs of transdermal patches that may be used, as shown in Figures 3-6, and discussed in more detail below. The particular patch design selected is determined based on a number of factors, one of the more important being the enhancer system. The simplest type of transdermal patch is the adhesive matrix patch where the drug and the enhancer are formulated into the skin adhesive layer. The patch consists of a backing material at the skin-distal side of the patch and a drug depot layer comprised of enhancer, drug, and adhesive which attaches directly to a patient's skin. Thus, the adhesive layer serves both as the drug and enhancer reservoir as well as adhesive. Adhesives by nature are lipophilic being comprised of long chain hydrocarbons. This allows for incorporation of most plasticizing type enhancers which are also typically lipophilic. On the other hand, many of the hydrophilic solvent type enhancers are not compatible with the adhesives. Because high flux rates of solvent enhancer (on the order of 0.1 mg/$cm^2 \cdot$hr or more) are required to achieve therapeutically effective flux rates of ketorolac, it is necessary to formulate patches having in the range of 50-100 mg of the solvent enhancer. Only a limited number of the hydrophilic solvent type enhancers typically used for transdermal patches can be formulated at these levels into an adhesive matrix. Solvent type enhancers which can be formulated into an adhesive matrix generally have a boiling point of greater than 100°C. One such enhancer, propylene glycol, is a preferred solvent type enhancer for the adhesive matrix patch embodiment of the present invention.

When solvents having a boiling point of less than 100°C are selected, the transdermal patch will generally be a monolithic matrix type or a reservoir type. These designs are discussed in further detail below. The matrix in these patches can be made from more hydrophilic materials in which the load of solvent enhancer can be contained along with the drug.

In general, the total amount of plasticizing enhancer will be less than the solvent enhancer. For example, a ratio of 1 part plasticizing enhancer to at least 10 parts solvent enhancer is preferred and more preferred is 1 part to 20 or greater parts solvent enhancer. A preferred combination for a reservoir-type device, described in detail below, is 20-60% of a $C_2$-$C_4$ alcohol enhancer, 0.5-20% of a plasticizing type fatty acid ester with the balance being water, glycerine, or propylene glycol. A most preferred combination for a reservoir device is 30-45% ethanol, and 1-5% isopropyl myristate with the balance being water. Ethanol in combination with isopropyl myristate at pH 7 is capable of increasing the skin penetration rate of ketorolac to therapeutically effective levels. This combination is advantageous because the use of a non-acidic enhancer formulation reduces irritation. In the adhesive patch it is possible to incorporate the lipophilic plasticizing type enhancers, such as the fatty acid ester isopropyl myristate. In Example 2 the use of propylene glycol, a solvent type enhancer, and isopropyl myristate is shown in a simple adhesive matrix type patch.

The adhesive used in an adhesive matrix type patch can be selected from a variety of adhesives available commercially and known to those in the art. For example, common adhesives are those based on poly isobutylene, acrylic, and silicone. The selection of the adhesive is critical to realize a functioning adhesive matrix type transdermal patch. The enhancers and the drug are loaded directly into the adhesive and so the adhesive must retain its functioning properties in the presence of these additives. These adhesive properties include sufficient tack for good instantaneous adhesion to the skin as well as maintenance of adhesion. Often adhesives become stringy and gooey in the presence of the skin permeation enhancers leading to cohesive failure and residual adhesive left on the patient's skin after removal of the patch. In some cases, the patch loses adhesion altogether and falls off. The loss of tack and other adhesion properties generally dictates and limits the amount and type of enhancers that can be loaded into the adhesive matrix type patches. Some acrylate based adhesives, such as those available from Avery and National Starch and Chemical company, are able to withstand relatively high loadings of enhancers, both solvent-type and plasticizing type.

An embodiment of the present invention is shown in Figure 3. Figure 3 shows a ketorolac adhesive matrix type transdermal patch, 1, which comprises an impermeable backing layer, 2, and an adhesive matrix layer, 3, which serves both as a depot for ketorolac and a means of adhering the device to the skin.

The impermeable backing layer, 2, defines the non-skin facing side of the patch in use. The functions of the backing layer include protection of the patch and the provision of an occlusive layer to prevent loss of ketorolac and the enhancers, in particular the solvent enhancer, to the environment. The material chosen should exhibit minimal ketorolac and enhancer permeability. The backing layer should be opaque because ketorolac degrades in the presence of light. Ideally, the backing material should be capable of forming a support onto which the ketorolac containing mixture can be cast and to which it will bond securely. A preferred material is polyester or aluminized polyester. Preferred backings are polyester medical films available for example from 3M Corporation such as Scotchpak® 1005 or 1109. As an alternative to casting the matrix directly on the backing, the polymer matrix may be cast separately and later stuck to the backing material.

The ketorolac adhesive matrix layer, 3, comprises ketorolac dispersed in an adhesive polymer matrix. As used herein, the term "dispersed" refers to the distribution of ketorolac throughout the matrix. The drug may be dispersed in a dissolved and/or undissolved state, but is preferably dissolved. The monolith layer may be prepared as follows. First a solution of the adhesive polymer is obtained or prepared. Another solution of ketorolac in appropriately selected enhancers is prepared and mixed until the drug is dissolved or evenly dispersed. The ketorolac/enhancer solution is then added to the adhesive polymer solution and the mixture is homogenized. The solution may be poured into a mold or cast alone or on the desired backing material. The casting is then left for the solvent to evaporate at room temperature or in an oven at a slightly elevated temperature. After solvent evaporation, the adhesive matrix takes the form of an adhesive polymer film typically having a thickness in the range of about 50 to 100 $\mu$m.

It will be appreciated that for a given desired total ketorolac load the percentage of loading may be varied by varying the adhesive matrix thickness. The total ketorolac content of the adhesive matrix will be sufficient to provide a one day dosage of ketorolac. Also the amount of ketorolac in the adhesive matrix may exceed the delivered load to keep the concentration gradient high so that the flux-rate of the patch remains constant throughout its intended use. For example, in a patch designed to deliver a total of 30 mg of ketorolac over a 24 hour period and then to be replaced by a fresh patch as much as 50 to 100 mg of ketorolac may be included in the patch. This ensures high activity of ketorolac at the end of the 24 hour period. For similar reasons excess plasticizing and solvent enhancers may also be included in the patch formulation.

A further embodiment of this invention describes reservoir type transdermal patches which may be used when very large amounts of solvent type enhancers are required. Typically, this type of patch is comprised of an impermeable backing layer which is sealed at its periphery to a rate-controlling membrane layer thus defining a drug depot. The drug depot generally contains the drug, and optionally an enhancer and/or gelling components. An adhesive layer on the rate-controlling membrane attaches the patch to the skin of a patient. A example of a reservoir type transdermal patch is provided in Example 8 where the two primary components of the formulation are ethanol and water. The patch comprises a backing layer and a porous membrane which are heat-sealed at their periphery to define a drug-depot.

The design of the reservoir type transdermal patch of the present application is effective in delivering ketorolac at flux rates of greater than 40 $\mu$g/cm$^2$·hr. Thus, the patches of the present invention can be used to deliver either the active enantiomer or the racemic form of ketorolac.

Looking at one basic embodiment of the present invention as shown in Figure 4, the ketorolac reservoir type transdermal patch, 4, comprises an impermeable backing layer, 2, and a drug depot (reservoir), 5 (which serves both as a depot for ketorolac and enhancer(s) used), a porous membrane, 6, and an adhesive layer, 7.

The impermeable backing layer, 2, defines the non-skin facing side of the patch in use. The functions of the backing layer are to provide an occlusive layer that prevents loss of ketorolac and the enhancers, in particular the solvent enhancer, to the environment and to protect the patch. The material chosen should exhibit minimal ketorolac and enhancer permeability. The backing layer should be opaque because ketorolac degrades in the presence of light. Ideally, the backing material should be capable of forming a support onto which the ketorolac containing mixture can be cast and to which it will bond securely. Preferred materials are aluminized polyester or polyester medical films available for example from 3M Corporation (Scotchpak® 1005 or 1109).

The membrane layer 6, and backing layer are sealed at their peripheral edges to form the drug reservoir, 5, which contains a solution or gel of the solvent enhancer and drug with plasticizing enhancer. As used herein, the term "peripheral edges" of the membrane and backing layers refers to the areas that are sealed together to define the drug reservoir. Therefore, extraneous membrane and backing layer material may extend outwardly from the drug reservoir and peripheral edge.

The contents of the reservoir may take various forms, for example, the ketorolac may be dispersed in an enhancer or combination of enhancers, gelled or ungelled. The drug may be dispersed in a dissolved and/or undissolved state, but is usually dissolved. Alternatively, the drug/enhancer mixture may be conveniently contained in the pores of a pad or foam material to keep the drug and enhancer in good contact with the membrane layer. Typically a polyurethane foam will be utilized because of its stability and permeability. The polyurethane may be of a polyether or polyester type. Polyether type polyurethanes are generally preferred, as they are more inert than polyester-type polyurethanes. Polymers of this type in various grades approved for medical use are commercially available. An example of a suitable polyurethane is the 1.8 inch polyurethane foam available from Foamex Corp.

In the transdermal patch of the present invention the membrane is extremely permeable to the enhancer and the drug contained in the reservoir. Thus the rate of permeation of the enhancer and drug through the membrane is high compared to their permeation rates through the skin. Generally, such a high flux rate of enhancer through the skin causes the skin to become irritated, thus requiring removal of the patch. This irritation problem can be overcome by diluting the enhancer with a non-irritating diluent such as water or other inert compounds such as mineral oil, silicon oil, and the like.

In some cases, when very powerful solvent enhancers are used, the diluted enhancer may be sufficiently effective in enhancing skin permeability to achieve the target flux rate for ketorolac (20-40 $\mu g/cm^2 \cdot hr$ for (-) ketorolac; 40-80 $\mu g/cm^2 \cdot hr$ for the racemate). Powerful solvent enhancers include N,N dimethylformamide, *n*-butyl acetate, or ethyl acetate. However, most solvent enhancers, when diluted to the point at which they are no longer irritating, are not effective in enhancing skin permeability to achieve the target flux rate for ketorolac. For example, if the solvent enhancer is ethanol, then a dilution with water to about 40 wt% ethanol is required before it becomes non-irritating; but 40 wt% ethanol is too dilute to achieve therapeutically effective flux rates of ketorolac.

The problem of using dilute solvent enhancers to increase the permeability of skin to ketorolac is overcome when a plasticizing-type enhancer is used in conjunction with the diluted solvent enhancer. When used in conjunction with a plasticizing type enhancer for the transdermal delivery of ketorolac, the solvent enhancer is generally loaded at levels of 2-20 $mg/cm^2$ of patch while much less plasticizing enhancer is used, typically 0.1 to 2 $mg/cm^2$, and more typically 0.5 to 1.0 $mg/cm^2$. The solvent type enhancer must be contained in the reservoir layer of the patch, but the plasticizing enhancer can be placed in the adhesive layer as well as the reservoir layer. The adhesive layer is a preferred location for placing the plasticizing enhancer because it is immediately adjacent to the skin. At this location, the plasticizing enhancer can immediately permeate into the skin producing a rapid enhancement effect. However, it can also be beneficial to place a portion of the plasticizing enhancer in the reservoir layer along with the solvent to minimize migration of the plasticizing enhancer into the reservoir layer during storage.

The nature of the membrane separating the reservoir from the skin is important. With transdermal devices of the prior art, the membrane layer separating the drug depot from the skin is generally a microporous membrane or a dense polymer film, that is one without a porous network. Dense polymer film can be fabricated from a wide variety of polymers such as ethylene vinyl acetate, silicone rubber, polyolefins, and the like. The polymer is selected based on the intrinsic permeability of the diffusing components in the polymer and the required flux rate of those components. This type of membrane is usually selected when it is desirable to control or limit the release of one of the reservoir compartments. Microporous membranes have a distinct pore structure with pores ranging in diameter from approximately 0.08 to 0.5 microns. Microporous membranes are commonly used in transdermal devices, especially the microporous polyethylene and polypropylene from 3M Company and from Hoescht-Celanese. Microporous membranes are useful when a lesser degree of control over release of the reservoir components is desired.

One would expect a microporous membrane with pores in the range of 0.2 to 0.5 microns to allow for adequate flux of the reservoir components as these pores are much larger than the size of the diffusing solvent and drug molecules. This can be shown with *in vitro* dissolution tests which reveal that microporous polyethylene releases the contents of the drug reservoir at a rate far higher than the skin's ability to absorb. However, when tested on skin, the flux of the drug is inadequate and below the desired target flux of greater than 40 $\mu g/cm^2 \cdot hr$. It may be that the flux of the solvent enhancer released from the microporous material is impeded due to some effect such as concentration polarization or the overall length to diameter ratio of the microporous membrane along any degree of tortuosity.

The membrane of the present invention is preferably not microporous in nature nor a dense polymer film. The transdermal patches of the present invention are prepared from a porous membrane material. As used herein, the term "porous membrane" refers to a membrane having pores greater than about 3 microns in diameter. Preferred porous membranes have pores greater than about 8 microns in diameter. Such materials are available as woven and non-woven fabrics, such as the non-woven polyester from Dexter Corp. (Windsor Locks, Connecticut). These materials can also be fabricated from nylon, polyethylene, and other polyolefins and the like. The improved fluxes provided from this material may be due to a convective wicking action of the fabric and/or to a high length to diameter ratio as these materials have pores in the range of 3-10 microns or more.

The adhesive layer used in the patch can be selected from a variety of adhesives available commercially and known to those in the art, as described above. The selection of the adhesive can be important to the proper functioning of the patch. This is particularly true if an enhancer is placed in the adhesive layer.

The liquid reservoir patch of the present invention will be useful in the transdermal delivery of other drugs (in addition to ketorolac) which require high flux rates of a solvent type enhancer to achieve a therapeutic effect.

Another type of transdermal patch is a monolithic matrix type where a material other than the adhesive serves as the drug depot. For these patches, hydrogel materials may be used such as polyurethane, gelatin, and pectin. Such materials can contain relatively large volumes of solvent type enhancer necessary for effective drug delivery. At the skin-facing side of the drug depot is a porous membrane layer, for example, a non-woven polyester membrane. The patch is attached to a patient's skin via an adhesive layer which is located at the skin-facing side of the membrane layer. The adhesive layer may cover the entire skin-facing side of the membrane, or may be located at the periphery of the membrane. Drug flux rates may be improved by placing plasticizing enhancer in the adhesive layer. These types of patches are disclosed in Examples 5, 6, and 7.

A monolithic matrix type transdermal patch according to the present invention is shown in Figure 5. Referring now to this figure, the ketorolac dispensing patch, 8 comprises an impermeable backing layer, 2, a monolithic matrix layer, 3, an optional porous membrane layer, 6, and an adhesive layer, 7. The backing, membrane, and adhesive layers are selected as described above for the embodiments of Figures 3 and 4. The porous membrane provides structural support for the adhesive layer which simplifies the manufacturing of the device. The monolithic matrix layer is distinguished from the adhesive matrix of Figure 3 where the monolith serves both as the drug reservoir and the skin adhesive.

In some instances, as shown in Figure 6, the adhesive layer 7, may be applied to the periphery of the patch so as not to come in contact with solvent enhancers. This is particularly desirable in situations where a high loading of solvent type enhancers may interfere with adhesion.

Preferred monolith matrix materials are those capable of holding a large volume of liquids, such as water and the chemical enhancers employed. Suitable materials are various polymers such as hydroxy ethyl methacrylate (HEMA) ethyl methacrylate (EMA) blends, polyvinyl alcohols, polyvinyl pyrrolidine, gelatin, pectin, and other hydrophilic materials. Microporous particles may be incorporated into the polymer monolith to hold the solvent type enhancers used. The use of microporous particles in transdermal patches is disclosed by Katz et al. in U.S. Patent No. 5,028,535, Sparks et al. in U.S. Patent No. 4,952,402, and Nuwayser et al. in U.S. Patent No. 4,927,687, all of which are hereby expressly incorporated by reference.

Ketorolac and enhancers may be loaded into the microporous particles before incorporation into the hydrophilic polymer. The particles are then evenly dispersed throughout the matrix by mixing. At high loadings of particles, the release of drug and enhancer is enhanced due to the formation of channels in the polymer matrix. Suitable microporous particles include diatomaceous earth, silica, cellulose acetate fibers from Hoechst Celanese, and Polytrap® from Dow Corning.

The solvent type enhancers used in a monolithic matrix or liquid reservoir transdermal patch are typically located in the drug reservoir. This is because of the hydrophilic nature of the solvent type enhancer which is generally present in relatively high proportions. The hydrophilicity makes it incompatible in the lipophilic adhesive layer so that incorporation of the enhancer system solely into the adhesive is difficult. The plasticizing enhancer used is more hydrophobic in nature and is more compatible with the adhesive. In one embodiment of both the reservoir and monolithic type patches, the plasticizing enhancer is located in the drug depot with the solvent enhancer and drug. In another embodiment, the plasticizing enhancer is incorporated into the adhesive layer while the solvent type enhancer and drug are located in the drug depot. Placement of the plasticizing type enhancer in the adhesive is often desirable because it puts the enhancer in direct contact with the stratum corneum. The solvent enhancer in the drug depot above the adhesive sweeps through the adhesive layer carrying the plasticizing enhancer with it into the skin. In some cases the plasticizing type enhancer is loaded into the adhesive as well as into the drug reservoir.

The invention is now further illustrated by Examples 1 to 9 which are exemplary but not scope-limiting.

## EXAMPLE 1

Skin permeation rates of various ketorolac/enhancer formulations were determined using flow through diffusion cells with an active area of 1 cm$^2$ and a receiving column of 3 ml. The receptor fluid, isotonic saline, was pumped into and through the cells by a peristaltic pump. Samples were collected in glass vials arranged in an automatic fraction collector. Human skin was placed on the lower half of the diffusion cell with the stratum corneum facing the donor compartment. The test solution or transdermal device was placed on the stratum corneum and the amount of drug permeated across the skin ($\mu$g/cm$^2$·hr) was calculated from the cumulative release.

## EXAMPLE 2

Transdermal patches with 10% ketorolac were prepared by the following procedure. Ketorolac, 0.5 g, was dissolved in 0.21 g of propylene glycol and 0.21 g of isopropyl myristate. This mixture was added to 12.5 g of a pressure sensitive acrylate adhesive solution (DT 9852 by National Starch, 34% solids content). The solution was left to mix for 12 hours on a rolling mill. The drug-enhancer-adhesive mixture was then cast on Scotchpak X-21220 polyester backing with a 1000 $\mu$m casting knife. The solvent in the adhesive solution was evaporated at room temperature for 30 minutes and then in an oven at 100°C for 15 minutes. The resulting adhesive film was laminated to a siliconized release liner (3M #1022). The resulting transdermal laminate was cut into disks 5 cm$^2$ for *in vitro* evaluation.

The average flux of ketorolac from this patch with 10% ketorolac, 8% enhancer (propylene glycol/isopropyl myristate), and 82% adhesive solids was 21.6 ± 3.59 $\mu$g/cm$^2$·hr. The average cumulative amount of ketorolac released after 24 hours from these systems was 461.7 ± 83.8 $\mu$g/cm$^2$.

## EXAMPLE 3

Patches were prepared according to the mixing and casting procedure of Example 2 except with the following amounts of components: 0.5 g of ketorolac, 0.25 g of propylene glycol, 0.25 g of isopropyl myristate, and 11.7 g of acrylate adhesive 34 wt% solution.

An *in vitro* skin flux experiment was conducted following the methods of Example 1. The average flux of ketorolac from this patch with 10% ketorolac, 10% enhancer, and 80% adhesive solids was $21.66 \pm 3.07$ µg/cm$^2$·hr. The average cumulative amount of ketorolac released after 24 hours from these systems was $324.26 \pm 88.63$ µg/cm$^2$.

**EXAMPLE 4**

Patches were prepared according to the mixing and casting procedure of Example 2 except with the following amounts of components: 0.5 g of ketorolac, 0.30 g of propylene glycol, 0.30 g of isopropyl myristate, and 11.4 g of acrylate adhesive 34 wt% solution.

An *in vitro* skin flux experiment was conducted following the methods of Example 1. The average flux of ketorolac from this patch with 10% ketorolac, 12% enhancer, and 78% adhesive solids was $36.56 \pm 5.35$ µg/cm$^2$·hr. The average cumulative amount of ketorolac released after 24 hours from these systems was $547.44 \pm 77.23$ µg/cm$^2$.

**EXAMPLE 5**

Monolithic ketorolac transdermal patches are prepared as follows. A solution of 44% ethanol, 22% glycerine, 22% propylene glycol and 12% ketorolac tromethamine is prepared. 11.5 g of the polyurethane prepolymer Hypol 5000 (WR Grace) is weighed into a plastic beaker. 5.0 g each of the above enhancer drug mixture and of Superfloss Diatomaceous Earth (Celite Corporation) is added to the Hypol 5000. After mixing well, the paste is cast between two sheets of siliconized polyester to a thickness of 540 µm on a double roll coater. The matrix is left overnight at room temperature to cure. After curing, a laminate of non-woven polyester and acrylate adhesive loaded with 30% isopropyl myristate is adhered to the matrix. Disks 5 cm$^2$ are cut from the matrix, and one side of the siliconized release liner is removed. The exposed matrix is placed on a sample of human skin and the permeation of ketorolac tromethamine is measured as described in Example 1.

**EXAMPLE 6**

A hydrogel-like ketorolac tromethamine transdermal matrix is prepared as follows. 3.0 g of glycerine and 7 g of deionized water are weighed into a beaker and mixed. 2.0 g of ketorolac tromethamine is added and mixed until dissolved. 10.0 g of 2-acrylamido-2-methylpropanesulfonic acid (AMPS, Lubrizol Corporation) is added and mixed. The mixture is purged with argon for 10 minutes while stirring. Then 0.5 g of 0.15% aqueous hydrogen peroxide is added and the prepolymer is cast onto Scotchpak X-21220 polyester backing. The prepolymer is cured in the oven at 45°C for 2 hours. After curing, the matrix is rehydrated in the presence of isopropanol, water, and ketorolac tromethamine (44:44:12). Disks 5 cm$^2$ are cut from the rehydrated matrix, and the permeation of ketorolac tromethamine is measured as described in Example 1.

**EXAMPLE 7**

Monolithic matrix ketorolac transdermal patches were prepared as follows. In a first container, ethanol (0.83 ml), deionized water (6.6 ml), glycerine (1.6 ml), and pectin (0.83 g) were mixed together and set aside. In a second container, 83 mg of potassium chloride was added to 1.66 ml of deionized water, mixed and set aside. In a third container, 1.3 g of ketorolac tromethamine was added to 6.6 ml of ethanol and mixed well. In a fourth container, gelatin (5 g), deionized water (5 ml) and glycerine (5 ml) were added together, mixed, and heated to 90°C with constant stirring. The melted gelatin mixture was added to the pectin component in the first container and mixed. The aqueous potassium chloride was then added, mixed, and then the ketorolac solution was added and mixed. The formulation resulted in a slightly viscous solution that was light amber in color. The solution was then cast onto a transdermal backing material. A laminate of adhesive film loaded with 30% isopropyl myristate and non-woven polyester (Dexter 9770) is placed over the matrix as it is cooling. As the matrix cools, it solidifies adhering the non-woven polyester/adhesive laminate to it.

The matrix was placed on stratum corneum and the release of ketorolac was measured according to Example 1. The average flux was $24.9 \pm 0.2$ µg/cm$^2$·hr. The average cumulative amount of ketorolac released after 24 hours from these systems was $555.2 \pm 187.8$ µg/cm$^2$.

**EXAMPLE 8**

An acrylate adhesive casting solution is prepared containing 30% isopropyl myristate. The casting solution is cast onto a polyester film (3M #1022) with a 750 µm knife and dried at room temperature for 30 minutes and then at 100°C for 15 minutes. A non-woven polyester membrane (Dexter Corp. #9770) is laminated to the resulting adhesive film. This three layer assembly is peripherally heat sealed to Scotchpak backing #1009 forming transdermal patches. The reservoir of the patch is filled with a 24% (-) ketorolac tromethamine solution in 37% ethanol, 35% water, 1.1% isopropyl myristate and 0.4% hydroxy propyl cellulose. The opening in the heat seal by which the fill solution is introduced is sealed after filling.

## EXAMPLE 9

An acrylate adhesive casting solution is prepared including 30% isopropyl myristate. The casting solution is cast onto a polyester film (3M #1022) and dried at room temperature for 30 minutes and then at 100°C for 15 minutes. A nonwoven polyester material (Dexter Corp. #9770) is laminated to the resulting adhesive film. This three layer assembly was peripherally heat sealed to 1/8 inch thick polyurethane foam (from Foamex Corp.) and a backing material (3M Corp, X-21220). The foam reservoir is filled with a 24% ketorolac tromethamine solution in 37% ethanol, 37% water, 1.1% isopropyl myristate and 0.4% hydroxy propyl cellulose. The opening in the heat seal by which the fill solution is introduced is sealed after filling and the patches are tested *in vitro* as described in Example 1.

The foregoing description of the preferred embodiments of the present invention has been presented for purposes of illustration and description. They are not intended to be exhaustive or to limit the invention to the precise form disclosed, and many modifications and variations are possible in light of the above teaching.

## Claims

1. A patch for the transdermal administration of ketorolac or (-) ketorolac capable of delivering therapeutically effective levels of ketorolac or (-) ketorolac through a patient's skin for a period of 12 hours or more, said patch comprising:

   (a) an occlusive backing layer having a skin-facing side; and

   (b) a drug depot having a skin-facing side and a skin-distal side in contact with said skin-facing side of said backing layer, said depot comprising:

      i. ketorolac or (-) ketorolac, and

      ii. an enhancer combination in which said ketorolac or (-) ketorolac is dispersed, said enhancer combination increasing the permeability of said ketorolac or (-) ketorolac through the stratum corneum and comprising a plasticizing-type enhancer and a solvent-type enhancer, said plasticizing type enhancer being a member selected from the group consisting of isopropyl myristate, caprylic triglyceride, capric triglyceride and glyceryl oleate, and said solvent type enhancer being a member selected from the group consisting of ethanol, propanol, propylene glycol, butanol and glycerin and

   said patch being capable of delivering, through said patient's skin, ketorolac at a flux rate of 40 $\mu$g/cm$^2$ hr or more, or (-) ketorolac at a flux rate of 20 $\mu$g/cm$^2$ hr or more.

2. A patch as claimed in claim 1 further comprising a porous membrane, optionally with pores greater than 8 microns in diameter, having a skin-facing side and a peripheral edge sealed to a peripheral edge of said backing layer and defining a drug depot therebetween, and an adhesive layer in contact with said skin facing side of said porous membrane.

3. A patch as claimed in claim 1 wherein said drug depot further comprises an adhesive polymer in which said ketorolac or said (-) ketorolac, and said enhanced combination are dispersed.

4. A patch as claimed in claim 1 further comprising an adhesive layer in contact with said skin-facing side of said depot, said patch optionally further comprising a porous membrane e.g. a non-woven polyester, having a skin-facing side and a skin-distal side in contact with said skin-facing side of said depot, said membrane being permeable to ketorolac or (-) ketorolac and said enhancer.

5. A patch as claimed in claim 2 wherein said porous membrane is a non-woven plastic.

6. A patch of claim 4 wherein a plasticizing-type enhancer is dispersed within said adhesive layer.

**7.** A patch of claim 4 or claim 6 wherein said polymer is a hydrogel.

**8.** A patch as claimed in any of claims 1 to 7 having an active surface area of less than 30 cm$^2$.

**9.** A patch as claimed in any of claims 1 to 8 wherein said enhancer combination comprises isopropyl myristate as the plasticizing-type enhancer.

**10.** A patch as claimed in any of claims 1 to 9 wherein said enhancer combination comprises a $C_2$-$C_4$ alcohol, e.g. ethanol or propylene glycol as the solvent type enhancer.

**11.** A patch as claimed in any of claims 1 to 10 wherein said depot further comprises a foam material, said ketorolac or (-) ketorolac and said enhancer combination being contained in said foam material.

**12.** The use of:

i. an occlusive backing layer having a skin-facing side, and

ii. means defining a drug depot having a skin-distal side in contact with said skin-facing side of said backing layer, said drug depot containing ketorolac or (-) ketorolac and an enhancer combination for increasing the permeability of said ketorolac or (-) ketorolac through the stratum comeum, said enhancer combination comprising a plasticizing-type enhancer and a solvent-type enhancer, said plasticizing type enhancer being a member selected from the group consisting of isopropyl myristate, caprylic triglyceride, capric triglyceride and glyceryl oleate, and said solvent type enhancer being a member selected from the group consisting of ethanol, propanol, propylene glycol, butanol and glycerin in the manufacture of a medicament in the form of a transdermal patch having a skin-facing side capable of delivering, through a patient's skin, ketorolac at a flux rate of 40 $\mu$g/cm$^2$ hr or more, e.g. to deliver less than 60 mg of ketorolac or less than 30 mg (-) ketorolac over a 24 hour period; optionally said patch having an active surface area of less than 30 cm$^2$.

**Patentansprüche**

**1.** Pflaster zur transdermalen Applikation von Ketorolac oder (-)-Ketorolac, welches therapeutisch wirksame Konzentrationen an Ketorolac oder (-)-Ketorolac über einen Zeitraum von 12 oder mehr Stunden durch die Haut eines Patienten abgeben kann, wobei das Pflaster umfasst:

(a) eine abschließende Rückenschicht mit einer der Haut zugewandten Seite; und
(b) ein Arzneimitteldepot mit einer der Haut zugewandten Seite und einer der Haut abgewandten Seite, in Kontakt mit der der Haut zugewandten Seite der Rückenschicht, wobei das Depot umfasst:

i. Ketorolac oder (-)-Ketorolac, und
ii. eine Verstärkerkombination, worin das Ketorolac oder (-)-Ketorolac dispergiert ist, wobei die Verstärkerkombination die Permeabilität von Ketorolac oder (-)-Ketorolac durch das Stratum corneum erhöht, und einen weichmachenden Verstärker und einen Lösungsmittel-Verstärker umfasst, wobei der weichmachende Verstärker ausgewählt ist aus der Gruppe bestehend aus Isopropylmyristat, Capryltriglycerid, Caprintriglycerid und Glyceryloleat, und der Lösungsmittel-Verstärker ausgewählt ist aus der Gruppe bestehend aus Ethanol, Propanol, Propylenglycol, Butanol und Glycerin, und

wobei das Pflaster Ketorolac mit einer Flussrate von 40 $\mu$g/cm$^2$h oder mehr, oder (-)-Ketorolac mit einer Flussrate von 20 $\mu$g/cm$^2$h oder mehr durch die Haut des Patienten abgeben kann.

**2.** Pflaster nach Anspruch 1, weiter umfassend eine poröse Membran, gegebenenfalls mit Poren mit einem Durchmesser größer als 8 $\mu$m, mit einer der Haut zugewandten Seite und einem peripheren Rand, der mit einem peripheren Rand der Rückenschicht verbunden ist und dazwischen ein Arzneimitteldepot begrenzt, und eine Klebeschicht, in Kontakt mit der der Haut zugewandten Seite der porösen Membran.

**3.** Pflaster nach Anspruch 1, worin das Arzneimitteldepot weiter ein Klebstoffpolymer umfasst, worin das Ketorolac oder (-)-Ketorolac und die Verstärkerkombination dispergiert sind.

**4.** Pflaster nach Anspruch 1, weiter umfassend eine Klebeschicht in Kontakt mit der der Haut zugewandten Seite des Depots, wobei das Pflaster gegebenenfalls weiter eine poröse Membran, z.B. einen non-woven Polyester umfasst, mit einer der Haut zugewandten Seite und einer der Haut abgewandten Seite, in Kontakt mit der der Haut zugewandten Seite des Depots, wobei die Membran für Ketorolac oder (-)-Ketorolac und den Verstärker durchlässig ist.

**5.** Pflaster nach Anspruch 2, worin die poröse Membran ein non-woven Kunststoff ist.

**6.** Pflaster nach Anspruch 4, worin ein weichmachender Verstärker innerhalb der Klebeschicht dispergiert ist.

**7.** Pflaster nach Anspruch 4 oder Anspruch 6, worin das Polymer ein Hydrogel ist.

**8.** Pflaster nach einem der Ansprüche 1 bis 7 mit einer aktiven Oberfläche, die kleiner ist als 30 cm$^2$.

**9.** Pflaster nach einem der Ansprüche 1 bis 8, worin die Verstärkerkombination Isopropylmyristat als den weichmachenden Verstärker umfasst.

**10.** Pflaster nach einem der Ansprüche 1 bis 9, worin die Verstärkerkombination einen $C_2$-$C_4$-Alkohol, z.B. Ethanol oder Propylenglykol als den Lösungsmittel-Verstärker umfasst.

**11.** Pflaster nach einem der Ansprüche 1 bis 10, worin das Depot weiterhin ein Schaummaterial umfasst, wobei Ketorolac oder (-)-Ketorolac und die Verstärkerkombination in dem Schaummaterial enthalten sind.

**12.** Verwendung von:

i. einer abschließenden Rückenschicht mit einer der Haut zugewandten Seite; und
ii. Mittel, die ein Arzneimitteldepot begrenzen, das eine der Haut abgewandten Seite besitzt, in Kontakt mit der der Haut zugewandten Seite der Rückenschicht, wobei das Arzneimitteldepot Ketorolac oder (-)-Ketorolac und eine Verstärkerkombination zur Steigerung der Permeabilität des Ketorolac oder (-)-Ketorolac durch das Stratum corneum enthält, wobei die Verstärkerkombination einen weichmachenden Verstärker und einen Lösungsmittel-Verstärker umfasst, wobei der weichmachende Verstärker ausgewählt ist aus der Gruppe bestehend aus Isopropylmyristat, Capryltriglycerid, Caprintriglycerid und Glyceryloleat, und der Lösungsmittel-Verstärker ausgewählt ist aus der Gruppe bestehend aus Ethanol, Propanol, Propylenglykol, Butanol und Glycerin, zur Herstellung eines Arzneimittels in Form eines transdermalen Pflasters mit einer der Haut zugewandten Seite, welche Ketorolac mit einer Flussrate von 40 μg/cm$^2$h oder mehr, z.B. weniger als 60 mg Ketorolac oder weniger als 30 mg (-)-Ketorolac über einen Zeitraum von 24 Stunden durch die Haut eines Patienten abgeben kann, wobei das Pflaster gegebenenfalls eine aktive Oberfläche von weniger als 30 cm$^2$ besitzt.

**Revendications**

**1.** Patch pour l'administration transdermique de kétorolac ou de (-) kétorolac, capable de délivrer des taux thérapeutiquement efficaces de kétorolac ou de (-) kétorolac au travers de la peau d'un patient sur une période de 12 heures ou plus, ledit patch comportant :

(a) une couche de protection occlusive ayant une face tournée vers la peau ; et

(b) un réservoir de médicament ayant une face tournée vers la peau et une face opposée à la peau en contact avec ladite face tournée vers la peau de ladite couche de protection, ledit réservoir contenant :

i. le kétorolac ou le (-) kétorolac, et

ii. une association promotrice dans laquelle ledit kétorolac ou (-) kétorolac est dispersé, ladite association promotrice augmentant la perméabilité dudit kétorolac ou (-) kétorolac au travers de la couche cornée et comportant un promoteur de type plastifiant et un promoteur de type solvant, ledit promoteur de type plastifiant était un composé choisi dans le groupe constitué du myristate d'isopropyle, du triglycéride caprylique, du triglycéride caprique et de l'oléate de glycéryle, et ledit promoteur de type solvant était un composé choisi dans le groupe constitué de l'éthanol, du propanol, du propylèneglycol, du butanol et de la glycérine, et

ledit patch étant capable de délivrer, au travers de lapeau dudit patient, le kétotolac à un débit de 40 $\mu$g/cm$^2$ par heure ou plus ou le (-) kétorolac à un début de 20 $\mu$g/cm$^2$ par heure ou plus.

2. Patch selon la revendication 1, comportant en outre une membrane poreuse, optionnellement avec des pores de diamètre supérieur à 8 microns, ayant une face tournée vers la peau et un bord périphérique scellé à un bord périphérique de ladite couche de protection et définissant un réservoir de médicament entre les deux, et une couche adhésive en contact avec ladite face tournée vers la peau de ladite membrane poreuse.

3. Patch selon la revendication 1, dans lequel ledit réservoir de médicament comporte en outre un polymère adhésif dans lequel sont dispersés ledit kétorolac ou ledit (-) kétorolac et ladite association promotrice.

4. Patch selon la revendication 1, comportant en outre une couche adhésive en contact avec ladite face tournée vers la peau dudit réservoir, ledit patch comportant optionnellement une membrane poreuse, par exemple un polyester non tissé, ayant une face tournée vers la peau et une face opposée à la peau en contact avec ladite face tournée vers la peau dudit réservoir, ladite membrane étant perméable au kétorolac ou au (-) kétorolac et audit promoteur.

5. Patch selon la revendication 2, dans lequel ladite membrane poreuse est un plastique non tissé.

6. Patch selon la revendication 4, dans lequel l'amplificateur de type plastifiant est dispersé au sein de ladite couche adhésive.

7. Un patch selon la revendication 4 ou la revendication 6, dans lequel ledit polymère est un hydrogel.

8. Un patch selon l'une quelconque des revendications 1 à 7, ayant une surface active inférieure à 30 cm$^2$.

9. Un patch selon l'une quelconque des revendieations 1 à 8, dans lequel ladite association promotrice comporte le myristate d'ïsopropyle à titre de promoteur de type plastifiant.

10. Un patch selon l'une quelconque des revendications 1 à 9, dans lequel ladite association promotrice comporte un alcool C$_2$-C$_4$, par exemple l'éthanol ou le propylène glycol, à titre de promoteur de type solvant.

11. Patch selon l'une quelconque des revendications 1 à 10, dans lequel ledit réservoir comprend une matériau de type mousse, ledit kétorolac ou (-) kétorolac et ladite association promotrice étant contenus dans ledit matériau de type mousse.

12. L'utilisation :

i. d'une couche de protection occlusive ayant une face toumée vers la peau, et

ii. d'un moyen définissant un réservoir de médicament ayant une face opposée à la peau en contact avec ladite face tournée vers la peau de ladite couche de protection, ledit réservoir de médicament contenant du kétorolac ou du (-) kétorolac et une association promotrice pour augmenter la perméabilité dudit kétorolac ou (-) kétorolac au travers de la couche cornée, ladite association promotrice comportant un promoteur de type plastifiant et un promoteur de type solvant, ledit promoteur de type plastifiant était un composé choisi dans le groupe constitué du myristate d'isopropyle, du triglycéride caprylique, du triglycéride caprique et de l'oléate de glycéryle, et ledit promoteur de type solvant était un composé choisi dans le groupe constitué de l'éthanol, du propanol, du propylène glycol, du butanol et de la glycérine, pour la fabrication d'un médicament sous forme d'un patch transdermique ayant une face tournée vers la peau capable de délivrer, au travers de la peau d'un patient, le kétorolac à un débit de 40 $\mu$g/cm$^2$ par heure ou plus, par exemple de délivrer moins de 60 mg de kétorolac ou moins de 30 mg de (-) kétorolac sur une période de 24 heures ; ledit patch ayant optionnellement, une surface active inférieure à 30 cm$^2$.

FIG. 1.

FIG. 2.

FIG. 3.

FIG. 4.

FIG. 5.

FIG. 6.